# EUROPEAN PATENT SPECIFICATION

(11) **EP 0 352 872 B1**
(45) Date of publication and mention of the grant of the patent: **24.04.1996**
(21) Application number: 89202513.1
(22) Date of filing: 24.05.1985
(51) Int. Cl.: A61B 17/22, A61B 17/32, A61M 29/02

(54) **Atherectomy device**
Vorrichtung für Atherektomie
Dispositif pour athérectomie

(30) Priority: 30.05.1984 US 615298; 10.05.1985 US 732691
(43) Date of publication of application: 31.01.1990
(62) Divisional of application: 85303693.7
(73) Proprietor: DEVICES FOR VASCULAR INTERVENTION INC., Palo Alto California 94304 (US)
(72) Inventor: Simpson, John B., Woodside California 94062 (US)
(74) Representative: Bucks, Teresa Anne

(56) References cited:
- EP-A- 0 086 048
- WO-A-82/00408
- DE-A- 2 804 015
- FR-A- 1 161 400
- GB-A- 2 044 103
- US-A- 3 811 448
- US-A- 3 990 453
- US-A- 4 273 128
- US-A- 4 290 427
- US-A- 4 295 464

## Description

This invention relates to an atherectomy device for removing or minimizing atheromas and for taking biopsies.

Peripheral vascular arteriosclerosis is a common ailment occurring in humans which involves the deposition of a fatty-like substance called atheromas or plaque in blood vessels and particularly in the peripheral blood vessels that feed the limbs of the human body. Occasionally these fatty deposits occur in fairly localised regions in the blood vessel. Substantial success has been obtained in increasing the size of the flow passages in the arteries in which these deposits have occurred by the use of a dilatation process using balloon angioplasty. However, it has been found that in a substantial percentage as, for example, 20 to 30% of the cases which are treated in this manner there is a tendency for the atheromas to reoccur. There is a need for a device which will substantially reduce such reoccurrences and thereby eliminate the need for additional dilitations.

FR-A-1161400 discloses an instrument for collecting biopsy specimens from the stomach and intestine comprising a cylinder formed with a side aperture and secured to a flexible sheath. The cylinder is provided with an annular cutter to which rotational and translation movement is imparted by a flexible tube connected thereto and which slides inside the flexible sheath. In use the apertured cylinder is placed under vacuum in order to suck material into the device before cutting.

US Patent No. 4273128 describes a device for the treatment of stenotic and occlusive coronary artery disease. A curved knife blade positioned at the distal end of the device is used to cut longitudinally through an obstruction in an artery. Thereafter, a balloon on an adjacent portion of the device is inflated to dilate the lumen of the artery.

The present invention provides an atherectomy device comprising a flexible member having proximal and distal ends; a relatively rigid cylindrical housing disposed at the distal end of the flexible member; said housing having a rounded distal end portion and a aperture on one side thereof; cutting means disposed within the housing; flexible drive means for translating the cutting means relative to the housing; and motive means for rotating the cutting means; characterised in that a continuous passageway extends through the flexible guide member and the housing for receiving a guide wire and to allow the guide wire to protrude out of the rounded distal end portion of the housing.

The following is a specific description of a number of preferred embodiments of the invention, reference being made to the accompanying drawings in which:
Figures 1A and 1B are side elevational views with certain portions broken away of an atherectomy device incorporating the present invention in which Figure 1A typically is approximately on a 10 to 1 scale and Figures 1B, except for the inflating device, typically is approximately on a 1 to 2 scale;
Figures 2A through 2E show how the atherectomy device shown in Figure 1 is utilised;
Figure 3 is a partial side elevational view with certain portions broken away of another embodiment of the atherectomy device incorporating the present invention;
Figure 4 is a cross section view taken along the line of Figure 3;
Figure 5 is a cross section view taken along the line 5-5 of Figure 3;

In general, the atherectomy device is comprised of a generally cylindrical housing formed of a relatively rigid material. The housing has rounded distal and proximal end portions. The housing is formed with a cutout extending longitudinally of the housing. Atheroma cutting means is disposed within the housing. Flexible guide means is secured to the proximal end of the housing for advancing the housing into the arterial vessel. Flexible drive means extends through the flexible guide means and is connected to the atheroma cutting means in the housing for causing operation of the atheroma cutting means to remove at least a portion of an atheroma from the arterial vessel.

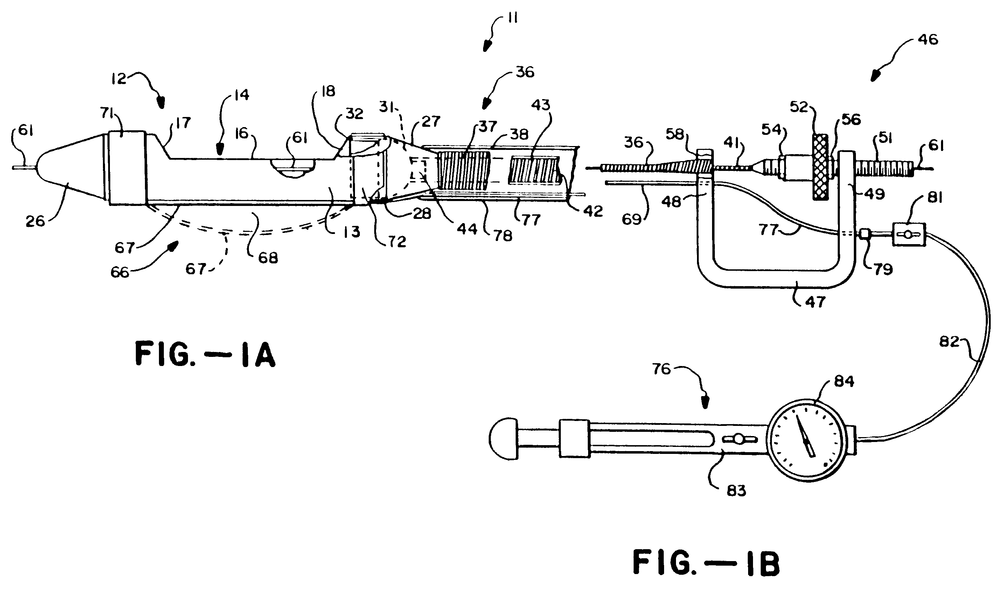

More particularly the atherectomy device 11 as shown in Figures 1A and 1B of the drawings consists of a housing 12 which has a generally cylindrical configuration. The housing 12 can be formed of any suitable relatively rigid materials as, for example, stainless steel. However, if desired a rigid plastic can be utilized, particularly if the device is to be disposable. The housing 12 consists of a cylinder 13 having a suitable diameter as, for example, ranging from 0.0762 cms to 0.381 cms (.030" to .150") The cylinder 13 is open-ended and is provided with a cutout 14 extending longitudinally of the same. The cutout 14 is formed so that the remaining portion of the cylinder in the vicinity of the cutout forms substantially a semi-cylinder. The cutout is defined by planar surfaces 16 of the sidewall of the cylinder lying in a plane parallel to the longitudinal axis of the cylinder 13 and upwardly and outwardly inclined end surfaces 17 and 18 adjoining opposite ends of the surfaces 16.

The housing 12 is provided with rounded end portions to facilitate movement of the housing within the coronary arteries. Thus on the distal extremity of the housing 12, there is provided a generally conical member 26 which is provided with a rounded extremity. The conical member 26 also is formed of a suitable material such as stainless steel or plastic. As shown the member 26 can be formed integral with the housing 12. Alternatively, it can be secured to the cylinder 13 by suitable means such as laser welding or a suitable adhesive.

A separate conical member 27 is secured to the proximal end of the cylinder 13. The proximal end of the cylinder 13 is provided with an annular recess 28 which is adapted to receive the conical member 27 and secured to the cylinder 13 by suitable means such as laser welding or by an adhesive. The housing 12 when so constructed has a smooth outer surface. The conical member 27 is truncated as shown.

Atheroma cutting means 31 is disposed within the housing 12. The cutting means 31 is cup-shaped as shown and is sized in such a manner so that it can fit into the cylinder 13 of the housing 12 and can be slidably moved longitudinally of the housing as hereinafter described. The cup-shaped cutting member 31 is provided with a circular continuous cutting edge 32 which lies in a plane perpendicular to the longitudinal axis of the housing 12. However, it should be appreciated that if desired the cutting edge can have other configurations. For example, the cutting edge can be inclined to form an ellipse. Also the cutting edge can be undulating if desired. The atheroma cutting means is inserted into the housing 12 before the conical member 27 is secured to the cylinder 13.

Flexible guiding means 36 is secured to the proximal extremity of the housing 12. The flexible guiding means 36 is in the form of an elongate hollow cylindrical cable-like member or coil spring 37 formed of a suitable material such as stainless steel. The cable-like member or spring 37 is covered with a tubular member 38 of suitable material such as a heat shrinkable plastic to prevent the coils of the cable-like member or spring from separating and also to enhance the type of control which can be provided by the cable-like member or spring so that the housing 12 and the flexible guiding means 36 can follow different curves in the blood vessels and tortuous segments of blood vessels. The flexible guiding means 36 can be secured to the truncated end of the conical member 27 by suitable means such as welding or an adhesive.

Flexible drive means 41 is provided for operating the cutting means 31 and is also formed of an elongate cylindrical cable-like member or coil spring 42 of a suitable material such as stainless steel and in which the cable-like member or spring 42 is covered with a tubular member 43 of suitable material such as a heat shrinkable plastic to prevent separation of the coils of the spring or cable 42 and also to facilitate movement of the flexible drive means 41 through the flexible guiding means 36. The flexible drive means 41 is secured to the proximal extremity of the atheroma cutting means 31 as shown particularly in Figure 1 by welding or brazing the spring onto a stud 44 forming a part of the cutting means 31. In certain applications it may be desirable to use a plastic tube formed of a suitable plastic such as Teflon (Registered Trade Mark) in place of the cable-like members used in the flexible guiding means 36 and the flexible drive means 41. Such a tube has been found to supply the necessary torsional strength.

Control means 46 is provided for controlling the movement of the housing 12 and operation of the cutting means 31 and for providing relative rotational movement between the same and movement of the cutting means 31 longitudinally of the axis of the housing 12. Such control means consists of a U-shaped control handle 47. The control handle is provided with spaced parallel legs 48 and 49. A threaded rod 51 is threaded into the leg 49 and extends in a direction at right angles to the leg 49. A knurled thumb screw 52 is rotatably mounted on the threaded rod 51 and is movable longitudinally of the rod and can be locked in a predetermined position by two lock nuts 54 and 56 adjustably positioned on the rod 51. The proximal extremity of the flexible guiding means 36 is mounted in a hole 58 provided in the leg 48 and is secured therein by a suitable means such as an adhesive. The flexible drive means 41 extends through the flexible guiding means 36 and has its proximal extremity secured to the tapered forward extremity of the threaded rod 51 by suitable means such as an epoxy. The knurled knob is positioned so that when the lock nut 56 is adjacent the leg 49, the cutting means 31 is in its rearmost extremity and when the lock nut 54 is in a position engaging the other leg 48, the cutting means 31 is in its forwardmost position. This ensures that the spring which serves as the flexible drive means 41 is not stressed unduly.

A guide wire of conventional construction 61 is provided which extends through a bore (not shown) provided in the threaded rod 51 and through the interior of the flexible drive means 41 and thence through the cutting means 31, through the housing 12 and through the conical member 26 of the housing 12.

Inflatable means 66 is provided for moving the housing 12 after it has been advanced into the desired position in the arterial vessel into close proximity to the atheroma on which a cutting operation is to be performed. The inflatable means 66 is carried by the housing 12 exterior of the housing 12 in a region opposite the cutout 14. This inflatable means 66 takes the form of a balloon 67 formed in a conventional manner integral with and at the end of an elongate tubular member 69 formed of plastic. The balloon can be very thin walled and need only take pressures typically in a range from 1.37 x 10⁵ to 2.055 x 10⁵ N/m² (20 to 30 psi). The balloon 67 preferably extends the length of the cylinder 13 and is secured to the exterior surface of the housing 12 at opposite ends of the cylinder 13 by suitable means such as bands 71 and 72 formed of a heat shrinkable plastic.

Inflating means 76 is provided for supplying a fluid to the balloon 67 and includes the flexible tubular member 77 which provides a lumen which opens into the balloon 67. The member 77 extends along the flexible guiding means 36 to the the control handle 47. It can be held in close proximity to the guiding means 36 by another heat shrinkable tubular member 78. The tubular member 77 is provided with a suitable fitting as, for example, a Luer adapter 79 which is adapted to be connected to suitable valve means as, for example, a two-way stop cock 81. The stop cook 81 is connected by a tube 82 to a balloon inflating device 83 called an "Indeflator" and described in United States Letters Patent No. 4,439,185. The Indeflator is used for introducing a fluid into the tubular member and to inflate the space 68 in the balloon 67 so that its outer side wall assumes the position represented. The pressure applied to inflate the balloon is observed by reading the gauge 84.

It should be appreciated that the tubular member 77, if desired, can be positioned within the flexible guiding means 36. Also, the U-shaped control handle 47 can be provided with openings or holes (not shown) in the arms 48 and 49 which can receive the tubular member 77.

Operation of the atherectomy device shown in Figures 1A and 1B may now be briefly described in conjunction with the method of the present invention as shown in Figures 2A to 2E. Let it be assumed that it is desired to cut out at least a portion of an atheroma in an arterial vessel. The arterial vessel 91 shown in Figure 2A has a wall 92 which has an atheroma 93 formed thereon. Let it also be assumed that it is desired to cut out at least a portion of the atheroma 93 in the arterial vessel 91. To accomplish this, an incision is made in the human body to make it possible to enter the arterial vessel in which the atheroma 93 is located. The distal tip of the guide wire 61 is inserted into the arterial vessel and advanced in the vessel until it extends through the arterial vessel through the stenosis, or narrowing 96 of the vessel created by the atheroma as shown in Figure 2A. Thereafter the proximal end of the guide wire 61 is threaded through atherectomy device 11 by inserting the guide wire 61 through the housing 12, the guiding means 36 and the threaded rod 51.

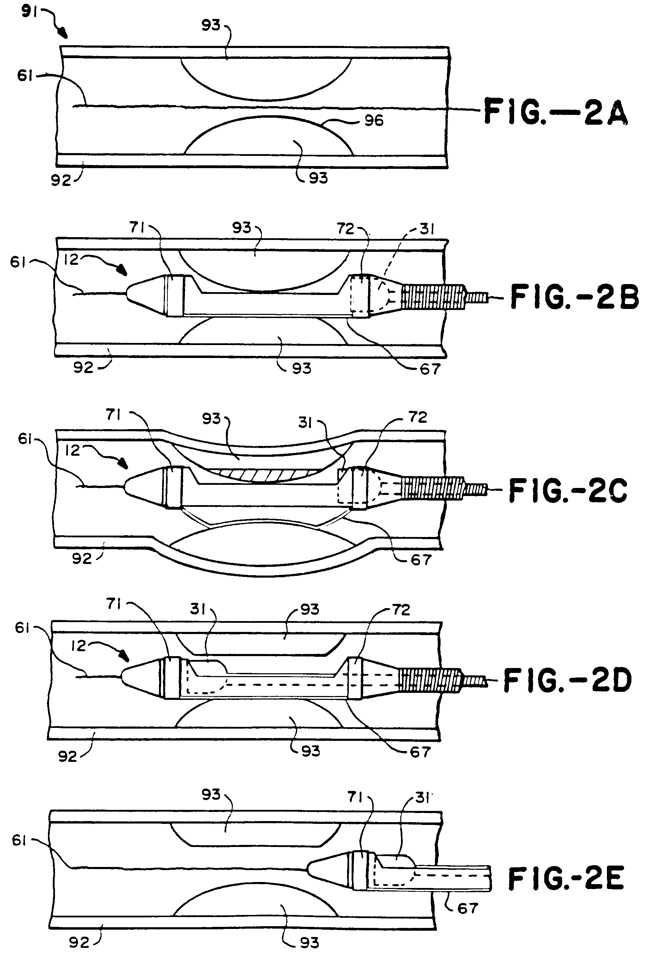

At this point in the operation, the cutting means 31 is completely retracted in the housing 12 to the position shown in Figure 1A of the drawings. The atherectomy device 11 is then inserted into the arterial vessel with the housing 12 going first. The flexible guiding means 36 provided for the housing 12 makes it possible for the atherectomy device to enter readily the arterial vessel and to follow the lumen in the arterial vessel. To facilitate advancement of the housing 12, the control handle 47 can be rotated back and forth. Advancement is continued until the housing 12 is positioned in the vicinity of the atheroma 93 as shown in Figure 2B. The positioning of the housing 12 can be observed by the use of a fluoroscope. The housing 12 as hereinbefore explained can be made of a material such as stainless steel which is relatively opaque to X-rays and thus gives a good image as the device is being positioned in the arterial vessel.

After the housing 12 is properly positioned as shown in Figure 2B, the balloon 67 is inflated by use of the inflating means 76 to force the housing 12 firmly into engagement with the atheroma 93 and particularly so that a substantial portion of the atheroma is positioned in the cutout 14. The inflation is accomplished by opening the stop cock 81 and introducing a fluid by operation of the Indeflator 83. The pressure at which the fluid is introduced into the balloon can be readily observed by reading the gauge 84 of the Indeflator 83. The balloon can be inflated with a saline solution or even air if desired since only a relatively low pressure is required, as for example, 1.37 x 10⁵ to 2.055 x 105 N/m² (20 to 30 psi). The inflation pressure is substantially less than that used for dilatation because the purpose is merely to position the housing 12 so it is in a snug position against the atheroma.

The balloon 67 when inflated engages the opposite side of the atheroma received by the cutout 14. As seen, the balloon 67 when inflated can accommodate the configuration of the atheroma 93 and thus apply a desired equalizing pressure to urge the housing towards the atheroma in the manner shown in Figure 2C. As this is occurring it has been found that the vessel wall 92 bulges outwardly below the balloon and bulges inwardly on the opposite side so that the atheroma is depressed into the cutout 14 of the housing 12 as shown in Figure 2C.

The cutting means 11 may now be operated. This is accomplished by rotating the thumb screw 52. As the thumb screw 52 is rotated as, for example, in a clockwise direction, the threaded rod 51 upon which the thumb screw 52 is mounted is rotated in the arm 47 so that the thumb screw 52 is advanced. This movement of thumb screw 52 causes rotation and advancement of the flexible drive means 41 which causes rotation and advancement of the cutting means 31. It should be appreciated that either or both the flexible guiding means 36 or the flexible drive means 41 can be rotated in opposite directions to cause this rotation with longitudinal advancement. The inflated balloon 67 serves to maintain the housing in engagement with the atheroma 93 so that it will not be pushed away from the atheroma 93 as the cutting means 31 engages the atheroma. This operation continues until the cutting edge 33 engages the atheroma and begins cutting the portion of the atheroma within the cutout 14 to remove the portion which is shown as cross hatched in Figure 2C. As the cross hatched portion is removed in the cutting operation in the manner shown in Figure 2D it is collected within the cup-shaped cutting means 31 and advanced into the forward conical-shaped extremity of the housing 12. The inflated balloon 67 serves to ensure that the atheroma 93 remains in the cutout during operation of the cutting means 31.

Thereafter, the entire atherectomy device 11 can be removed from the arterial vessel as shown in Figure 2E taking with it the portion of the atheroma which has been severed. After the device has been removed from the arterial vessel, the material collected within the housing 12 is removed. In removing the atherectomy device, the guide wire can be left in place so that multiple cutting operations can be performed if necessary to remove sufficient material from the atheroma. If multiple cutting operations are to be performed on the same atheroma, the device can be removed after each cutting operation and cleaned. Additional portions of the atheroma can be removed with additional passes of the cutting means 31 until a sufficiently large passage is established to ensure that there is very little likelihood of any further possibility of occlusion of the blood vessel.

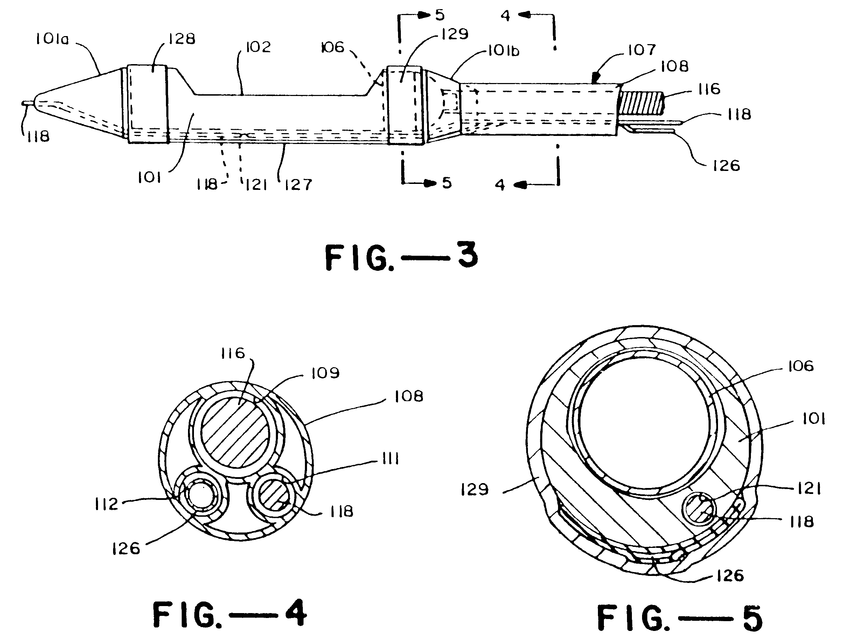

Another embodiment of the atherectomy is a device is shown in Figures 3 through 5. As shown therein, it also consist of a cylindrical housing 101 which is formed of plastic and which is provided with an elongate cutout 102 similarly to the cutout 14 hereinbefore described. The distal extremity of the housing is provided with a rounded portion 101a and similarly the proximal extremity of the housing 101 is provided with a rounded portion 101b. This rounded portion 101b can be formed as a separate part and bonded to the main body of the housing 101 by suitable means such as an adhesive after the atheroma cutting means or cutter 106 has been mounted within the cylindrical housing. The cutter 106 can be identical to the atheroma cutting means 31 hereinbefore described and is movable longitudinally of the housing 101.

Flexible guiding means 107 is secured to the proximal extremity of the conical portion 101b of the housing 101 and consists of a flexible plastic tube 108. The tube 108, as shown, can be formed as an extruded member which is provided with three separate lumens 109, 111, and 112.

Flexible drive means in the form of a cable 116 is provided for driving the cutter 106. The cable 116 can be hollow or solid. It is shown as being solid in Figure 4 and is disposed in the large lumen 109 provided in the tube 108. The distal extremity of the flexible cable 116 can be secured to the cutter 106 by a suitable means such as an adhesive. The flexible tube 108 can be secured to the leg 48 of the U-shaped member 47 in the same manner that the guiding cable 36 is secured thereto. Similarly, the drive cable 116 can be secured to the knob 52 in the same manner that the flexible drive means 41 is secured to the knob 52. A flexible guide wire 118 is provided which extends through the lumen 111. The lumen 111 is in communication with a passageway 121 which is provided in the plastic housing 101. The passageway 121 extends the length of the housing 101 and curves up towards the distal extremity of the same so that the passage 121 exits centrally of the housing 101. As can be seen, the guide wire 118 extends through this passageway 121 and exits through the rounded portion 101a so that it can be utilized for guiding the housing 101 in its travel through the arterial blood vessel.

A balloon tube 126 extends through the lumen 112 and is provided with an integral inflatable balloon 127 of the same type as balloon 67. The inflatable balloon 127 is secured to the housing 101 by plastic bands 128 and 129.

The proximal extremities of the guide wire 118 and the balloon tube 126 can be brought out at the proximal extremity of tube 108 adjacent the U-shaped member 47. The balloon tube 126 can be connected to an inflating device such as device 76 as shown in Figure 1B. The guide wire 118 can be manipulated in a conventional manner.

Operation of this embodiment of the atherectomy device shown in Figures 3 through 5 may now be briefly described as follows. The operation of the device is very similar to that hereinbefore described with respect to the embodiment as shown in Figures 1A and 1B. The principal difference between the two embodiments is that the guide wire 118 need not pass through the cutter 106 or through the center of the housing, as is the case, in the embodiment shown in Figures 1A and 1B. Rather, as shown, the guide wire is introduced through the housing through a passageway 121 provided in the housing. This makes it possible to insert and remove the guide wire 118 from the atherectomy device even though material may already have been cut by the cutter 106 and be disposed within the housing 101.

It is apparent from the foregoing that there has been provided an atherectomy device which is particularly efficacious in removing material from atheromas in arterial vessels. This is particularly advantageous in that it is less likely that such atheromas will reoccur after they have been removed surgically by the device of the present invention utilising the present method.

## Claims

1. An atherectomy device (11) comprising a flexible member (36,107) having proximal and distal ends; a relatively rigid cylindrical housing (12,101) disposed at the distal end of the flexible member (36,107); said housing (12,101) having a rounded distal end portion (26,101a) and a aperture (14,102) on one side thereof; cutting means (31,106) disposed within the housing (12,101); flexible drive means (41) for translating the cutting means (31,106) relative to the housing (12,101); and motive means (51,52) for rotating the cutting means (31,106); characterised in that a continuous passageway extends through the flexible guide member (36,107) and the housing (12,101) for receiving a guide wire (61,118) and to allow the guide wire to protrude out of the rounded distal end portion (26,101a) of the housing (12,101).

2. An atherectomy device as claimed in Claim 1, wherein the passageway extends through the centre of the housing (12).

3. An atherectomy device as claimed in Claim 1, characterised in that the passageway (121) through the housing (101) is disposed along a side of the housing (101).

4. An atherectomy device as claimed in Claim 3, characterised in that the passageway (121) curves near the distal end of the housing (101) and exits the housing (101) through the centre of the rounded distal end portion (101a).

5. An atherectomy device as claimed in any preceding claim in combination with a guide wire (61,118).

## Patentansprüche

1. Vorrichtung (11) zur Atherektomie, umfassend: ein flexibles Element (36, 107) mit einem proximalen und einem distalen Ende; ein relativ starres zylindrisches Gehäuse (12, 101), das an dem distalen Ende des flexiblen Elements (36, 107) angeordnet ist; wobei das Gehäuse (12, 101) einen abgerundeten distalen Endabschnitt (26, 101a) und an seiner einen Seite eine Öffnung (14, 102) aufweist; ein Schneidmittel (31, 106), das in dem Gehäuse (12, 101) angeordnet ist; ein flexibles Antriebsmittel (41) zum Verlagern des Schneidmittels (31, 106) relativ zu dem Gehäuse (12, 101); und ein Bewegungsmittel (51, 52) zum Drehen des Schneidmittels (31, 106), **dadurch gekennzeichnet,** daß eine durchgehende Passage durch das flexible Führungselement (36, 107) und das Gehäuse (12, 101) führt, um einen Führungsdraht (61, 118) aufzunehmen und es dem Führungsdraht zu ermöglichen, aus dem abgerundeten distalen Endabschnitt (26, 101a) des Gehäuses (12, 101) vorzustehen.

2. Vorrichtung zur Atherektomie nach Anspruch 1, in der die Passage durch die Mitte des Gehäuses (12) verläuft.

3. Vorrichtung zur Atherektomie nach Anspruch 1, dadurch gekennzeichnet, daß die Passage (121) durch das Gehäuse (101) längs einer Seite des Gehäuses (101) angeordnet ist.

4. Vorrichtung zur Atherektomie nach Anspruch 3, dadurch gekennzeichnet, daß die Passage (121) nahe dem distalen Ende des Gehäuses (101) gekrümmt ist und aus dem Gehäuse (101) durch die Mitte des abgerundeten distalen Endabschnitts (101a) austritt.

5. Vorrichtung zur Atherektomie nach einem der vorhergehenden Ansprüche in Kombination mit einem Führungsdraht (61, 118).

## Revendications

1. Appareil d'athérectomie (11) comprenant un organe flexible (36, 107) ayant des extrémités proximale et distale, un boîtier cylindrique relativement rigide (12, 101) disposé à l'extrémité distale de l'organe flexible (36, 107), le boîtier (12, 101) ayant une partie arrondie d'extrémité distale (26, 101a) et un orifice (14, 102) d'un côté, un dispositif de coupe (31, 106) placé dans le boîtier (12, 101), un dispositif flexible (41) d'entraînement destiné à déplacer en translation le dispositif de coupe (31, 106) par rapport au boîtier (12, 101), et un dispositif moteur (51, 52) destiné à faire tourner le dispositif de coupe (31, 106), caractérisé en ce qu'un passage continu est disposé dans l'organe flexible de guidage (36, 107) et le boîtier (12, 101) afin qu'il loge le fil de guidage (61, 118) et permette au fil de guidage de sortir de la partie arrondie d'extrémité distale (26, 101a) du boîtier (12, 101).

2. Appareil d'athérectomie selon la revendication 1, dans lequel le passage est disposé au centre du boîtier (12).

3. Appareil d'athérectomie selon la revendication 1, caractérisé en ce que le passage (121) formé dans le boîtier (101) est placé le long d'un côté du boîtier (101).

4. Appareil d'athérectomie selon la revendication 3, caractérisé en ce que le passage (121) se recourbe près de l'extrémité distale du boîtier (101) et quitte le boîtier (101) au centre de la partie arrondie d'extrémité distale (101a).

5. Appareil d'athérectomie selon l'une quelconque des revendications précédentes, combiné à un fil de guidage (61, 118).
